# EUROPEAN PATENT APPLICATION

(11) **EP 4 138 086 A1**
(43) Date of publication of application: **22.02.2023**
(21) Application number: 21191450.2
(22) Date of filing: 16.08.2021
(51) Int. Cl.: G16H 20/00, G16H 40/60

(54) **COMPUTER-ASSISTED IMAGING USER GUIDANCE**

(71) Applicant: Koninklijke Philips N.V., 5656 AG Eindhoven (NL)
(72) Inventor: VOGTMEIER, Gereon, 5656 AE Eindhoven (NL); SISODIA, Rajendra Singh, 5656 AE Eindhoven (NL); LUESSLER, Christoph Günther, 5656 AE Eindhoven (NL); WEISS, Steffen, 5656 AE Eindhoven (NL); JOHNSON, Mark Thomas, 5656 AE Eindhoven (NL)
(74) Representative: Philips Intellectual Property & Standards

(57) **Abstract**

A user guidance system (SYS) and related method The system comprises an interface (IN) for receiving a specification of a medical protocol for a given patient, the specification defining a sequence of medical action points. A synthesizer (S) of the system is configured to synthesize a media sequence in accordance with the sequence of medical action points, to obtain a synthesized media sequence for the said patient, the media sequence configurable to guide the patient in a medical procedure, based on the protocol. The media sequence may be used to support imaging protocols to encourage patient compliance, and hence better imaging for example.

## Description

### FIELD OF THE INVENTION

The invention relates to a user guidance system, to an arrangement using such as system, to a computer-implemented method of user guidance, and to a related computer program element and computer readable medium.

### BACKGROUND OF THE INVENTION

Modern medicine relies on a range of expensive and delicate equipment and machinery. For example, being able to quickly obtain good quality diagnostic imagery of internal tissue/organs of a patient lies at the heart of most branches of the medical profession. Imaging systems are used for this, such as an X-ray imaging system or an MRI system, or others still.

Operating such medical imaging systems safely and usefully, requires great skill. A range of complex settings or adjustments may need to be made based on machine control parameters to ensure the outcome of a given medical procedure (such as imaging) is useful and successful.

Recently, in order to increase patient throughput or efficiencies in general, at least partly autonomized/robotized imaging equipment was proposed, with little or no on-site involvement by medical staff.

However, the "machine side" of a successful/useful medical procedure is only one aspect. Another, equally important one is patient compliance, and in particular so in autonomous settings: in some medical procedures, the patient will need to perform certain actions, correctly and in the right order before, during and/or after the medical procedure. This can be challenging and error-prone, especially during longer such procedures. For example: the patient is asked to avoid any movement at defined points of time, or should breathe with a defined timing, etc. Or the patient may be asked to interact at defined imaging sequences, such as in MRI.

For the patient, in general not medically trained, it may not be easy to understand, let alone remember, the requisite action points in particular during a long imaging sequence where multiple images are acquired at different patient postures for example. In addition, the exact timing in combination with other supportive patient actions may be hard to remember and get right first time. There may also be a sequence of action points expected of patient before and after the actual imaging acquisition.

High patient throughput in busy clinics, the need for good imagery, and the desire to reduce wear-and tear on expensive medical equipment may be difficult objectives to reconcile at the same time. This is all the more true in the above mentioned (at least partly) autonomized/robotized imaging settings. Fostering patient compliance can help in achieving all three objectives.

### SUMMARY OF THE INVENTION

There may be a need to address at least some of the above-mentioned challenges. In particular, there may be a need for improved patient guidance, in particular in relation to medical procedures.

An object of the present invention is achieved by the subject matter of the independent claims where further embodiments are incorporated in the dependent claims. It should be noted that the following described aspect of the invention equally applies to the arrangement, the computer-implemented method, the computer program element and to the computer readable medium.

According to a first aspect of the invention there is provided a user guidance system (SYS) comprising:
an interface (IN) for receiving a specification of a medical protocol for a given patient, the specification defining a sequence of medical action points;
a synthesizer (S) configured to synthesize a media sequence in accordance with the sequence of medical action points, to obtain a synthesized media sequence for the said patient. The media sequence may be used to guide the patient in a medical procedure, based on the protocol. The guidance may be during the medical procedure and/or prior to commencement of the medical procedure.

The media sequence may include audio, video, multimedia, etc.

The medical protocol may be medical imaging protocol for an imaging session with certain imaging modalities, such as MRI, X-ray CT, etc. In addition, other types of medical protocols may be supported with the proposed system.

In embodiments, the media sequence is of a pre-selected category.

The category may include type/genre of movie, type of story (action, crime, etc), or category could include also "*roadtrip*, *biketrip*, ...", travelogue, road trip movie, etc. The type of movie, type of story, etc may be part of the category.

In embodiments, the system comprises a selector, configured to select the category based on i) input from the patient or user received through a user interface, and/or ii) based on characteristics of the patient.

In embodiments, the system of comprises a transducer controller configured to control, based on the synthesized media sequence, a transducer to produce a media output for the patient. Transducer may include a speaker system and/or display device, a haptics generator, a VR/AR head-set, etc.

In embodiments, the synthesized media sequence comprises a sequence of media elements associated with respective ones of the action points. The media elements may represent different scenes, or portions of story, movie, plot, etc.

In embodiments, the synthesizing operation includes: i) changing an order of media elements in an existing media sequence and ii) modulating a media element in accordance with an action point.

Modulation may include changing a color case during replay, and or changing rhythm, mode or any other musical characteristics of a sound-track. A quicker rhythm may motivate the patient to perform the action point faster. A slower rhythm may be helpful to motivate patient for the action point to be performed slower. The patient may be induced to change their breathing frequency, etc. Modulation may include selection of actions or integration of additional special action scenes in the media sequence.

In embodiments, the system comprises a delay measurement device configured to measure an elapsed time period between the transducer being controlled as per a given media element and the patient preforming the associated action point.

In embodiments, the system comprises a tuner module, configured to tune the specification of the medical protocol based on the measured time period to produce a tuned specification of the medical protocol.

In embodiments, the system comprises a control interface, configured to control a medical imaging apparatus or therapy apparatus, based on the tuned specification.

In embodiments, the media sequence is one or more of: audio, imagery and video.

In embodiments, the synthesizer is based on a machine learning model of the generative type.

In another aspect there is provided an arrangement, comprising a medical imaging or therapy device and the system as per any one of the above mentioned embodiments.

In another aspect there is provided a computer-implemented method, comprising:
receiving a specification of a medical protocol for a given patient, the specification defining a sequence of medical action points; and
synthesize a media sequence in accordance with the sequence of medical action points, to obtain a synthesized media sequence for the said patient, the media sequence configurable to guide the patient in a medical procedure, based on the protocol.

The method may further comprise playing back or replaying the synthesized media sequence. As display device and/or an acoustic transducer, or any other suitable player/transducer equipment may be used.

The medical action points may include suitably encoded instructions for the patient to assume a certain posture, move in a certain manner, remain stationary for a given time period, breath at a certain frequency, hold their breath, or any other action the patient is required to perform during the medical procedure. For example, the medical action points may include instructions for moving the left/right arm in a certain manner, moving a leg, moving their head from one position to another, etc. The medical action points may be configured to support the medical procedure so as to facilitate a successful conclusion thereof, such as obtaining a result (imagery) of sufficient image quality, etc.

Because the media sequence as synthesized herein is tailored around patient's preference for a given media category, the patient can better remember and execute the asked of medical action points. The media sequence synthesized herein can induce a high level of patient compliance. The high level of compliance may translate into correct and timely conclusion of the medical procedure. For example, quicker imaging sessions may be conducted that still result in diagnostic imagery for example, and that put less strain on patient and/or equipment, etc.

The media sequence may include video footage and/or an audio sequence. The media sequence may represent a story, a plot, etc, featuring preferred actor(s), character(s), or being of a preferred genre, being by a preferred author, director, etc.

The media elements of the media sequence my include frames of groups of such frames representing respective scene(s). The scenes are a representation of a desired action point. The media sequence may include plural such media elements for plural scenes for example. The media sequence may thus represent a sequence of action points as per a protocol. The synthesized media sequence, once played back or replayed on a player component, represents the requisite sequence of action points as per a predefined imaging protocol for example. Thus, the action points are embedded as "landmarks" in a media sequence context of a category preferred by the patient. The so synthesized "memory map", that is the synthesized media sequence, fosters high memory retention in patient, and thus high compliance and quicker and successful conclusion of the medial procedure.

The medical procedures envisaged herein may include an imaging session (such as using X-ray, MRI or other modality). The medical protocol may include an imaging protocol. Alternatively, the medical procedure may be one of a radiation therapy delivered by a linac for example, to treat tumors. The medical protocol may thus involve an RT treatment protocol, for example.

"*User*" and "*patient*" are mainly used interchangeably herein. The patient/user is in general the person who takes part in the medical procedure It is the person for whom the procedure is run. When reference is made herein to "medical personnel" (so not the user/patient), this relates to person(s) who operate the imaging apparatus or who oversee the imaging procedure, or those who may process the outcome of the procedure. Such persons may also be referred to herein as "medical user", "staff', "personnel", etc.

### BRIEF DESCRIPTION OF THE DRAWINGS

Exemplary embodiments of the invention will now be described with reference to the following drawings, which are not to scale, wherein:

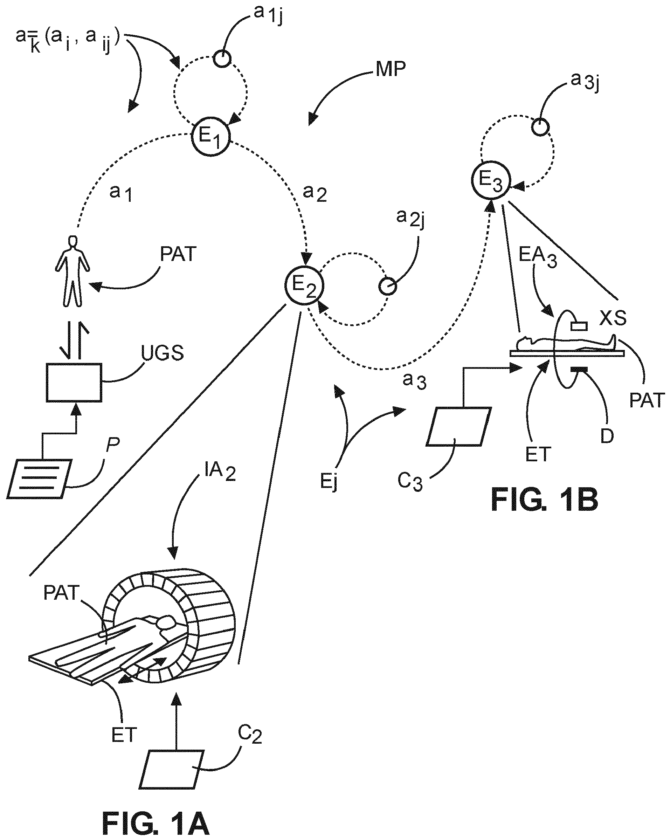

Fig. 1 shows a schematic illustration of a medical procedure including action points to be performed by a patient taking part in the said procedure;
Fig. 1 2 shows a schematic block diagram of a computer implemented system for user guidance during and/or prior to a medical procedure; and
Fig. 1 3 shows a flow chart of a computer-implemented method for user guidance during and/or prior to a medical procedure.

### DETAILED DESCRIPTION OF EMBODIMENTS

Before explaining operation of the proposed system and method, reference is first made to Fig. 1, in order to illustrate context in which the proposed system and method may be practiced. Specifically, Fig. 1 is a schematic diagram of a medical procedure MP in which a patient PAT is expected to participate. Briefly, and as will be expanded upon in more detail below (at Figs. 2,3), a computer implemented guidance system is proposed herein for use by a patient to better implement the medical procedure with respect to patient PAT.

For example, medical procedures mainly envisaged herein are imaging sessions and/or radiotherapy sessions or others still. The proposed context may be one for diagnostic and/or therapeutic purposes. For illustration, main reference will be made herein to medical imaging sessions, but this is not at the exclusion of other types of medical procedures.

The proposed system and method are configured herein to support such medical procedures in a preferably autonomous setting, such as autonomous imaging environments in a clinic or other medical facility for example. In such autonomous settings, the patient is expected to take part in the medical procedure without or with only limited guidance from human medical personnel on-site. The medical procedure may be administered at least partly (if not fully) by at least partly (if not fully) automized or robotized equipment Ej. The patient may be asked to visit one or more of items of such medical equipment Ej, and is expected to perform respective one or more medical actions, referred to herein as medical action points aj, at, or in relation to, the said respective item of medical equipment Ej.

For example, the medical equipment Ej may include one or more imaging apparatus ("imager" for short) IA2, IA3, such as is illustrated in in-inset Figs. 1A, 1B as equipment E2,E3. Operation of imager(s) may be at least partly automated. Operation of the imager IA2, IA3 may be controlled by providing control parameters cj as may be prescribed in an imaging protocol P.

Imager IAI2 may be a magnetic resonance imaging ("MRI") apparatus. The control parameters C2 may control interaction of magnetic fields and electric fields generated by magnets and radio frequency coils of the imaging apparatus. Patient PAT may be expected to lie on an examination table ET. The examination table ET with the patient on it is introduced into an examination region, formed by an opening, a bore, of the imager IA2. Based on the control parameters C2, coil circuitry is operable to generate radio RF pulses that interact with patient's tissue and the generated magnetic field that pervades, in particular, the bore. The interaction causes response radio frequency signals which are detected by the same and/or other coil circuitry of the imager IA2 to produce sectional images that may represent internal structures (tissue, organs, anatomies) of patient PAT.

Another, or additional type of medical equipment E3 may include an x-ray imaging apparatus IA3. The patient may or may not lie on an examination table ET, in an examination region between an x-ray source XS and, opposed thereto, an x-ray detector D. Based on control parameters C3, the imager IA3 is operable to expose patient PAT to X-ray radiation emanating from the x-ray source XS. The x-radiation interacts with patient tissue to produce modified radiation. The modified radiation is detected by the detector D. Acquisition circuitry converts the detected radiation into x-ray imagery. The x-ray imager IA3 may be of the radiography type to produce projection imagery, or may be of the tomographic type where the x-ray source rotates around the patient to acquire projection imagery from different directions which can then be reconstructed into cross-sectional imagery in one or more planes to form an image volume, if required.

Imaging equipment AI3 and AI2 may be robotized. For example, the introduction of table/patient into the bore, or the operation of the x-ray source etc is done by actuators communicatively coupled to a surveillance system (not shown), such as video cameras that monitor the patient by collecting surveillance data such as imagery or video. Based on the data collected during monitoring operation, the actuators are adjusted accordingly to effect introduction into the bore of the patient, and or/movement of source XS, or of any other mode of operation of components of the medical imaging equipment IA2, 3.

Imagers other than X-ray or MRI are also envisaged herein, such as those of the emission type (nuclear imaging), ultrasound ("US"), or others still. However, the principles described herein are not necessarily confined to imaging. Other type(s) of medical equipment Ej may be used instead or in addition to imager(s), at different phases of the medical procedure. For example, the said other types of equipment E1 may include other, preferably robotized/automated systems, such as ones configured to measure patient's blood pressure, heart rate, oxygenation, or other vital signs. For example, some type of robotic equipment Ej may be operable to collect a blood sample from patient, to administer medication and/or other substances such as contrast agents etc. The equipment E1 may include surgical robots, and others still. For example, in a radiation therapy context, the equipment Ej may include a radiation therapy delivery apparatus, such as a linear accelerator("linac").

As briefly mentioned earlier, the representation of the medical procedure with patient participation as per Fig. 1 is schematic and illustrative: for example, a single imager may be used instead of two or more as shown. Indeed, no imager may be used at all in some medical procedures. A single item of medical equipment Ej may be used in some medical procedures. A combination, not necessarily at the same time, of different types of medical equipment may be used in others. The medial procedure may include "loops", where one and the same equipment is reused or revisited at different phases of the medical procedure, inviting the same, similar or different action points. Such loops are optional however. The same equipment may be used more than once, inviting different action points at different times, or repetitions. The patient PAT may be required to move from one equipment Ej to another Ej+1, the two items of equipment Ej, Ej+1 being spatially remote from each other. Thus, the patient may need to make their way through the medical facility, possibly from one room to another. However, such medical perambulation may not be required in some procedures or setups, and all action points are carried out in the same physical location, such as in the same exam room, using one or more items of equipment at phases of the procedure. If such perambulation is required, one or more of the action points may then represent the patient's movement from one equipment to the next. Alternatively, the movement from one equipment to other is not accounted for as a medical action point. More than one item of medical equipment may be used at a given time. At other times or procedures, a single equipment is used at a given time, for at least some instances.

In medical procedures in an autonomous setting, very little on-site involvement of medical personnel may be required, if any. If at all, human user may be involved through remote telecommunication to at least partly control at least some phases of the procedure from a remote location for example. For example, this may allow a single surgeon or imaging technician to conduct multiple sessions remotely in telemedicine sessions, thus improving patient throughput. In extreme cases, there is no medical staff/personnel on-site at all. Medical procedures can thus be conducted with fewer medically trained personnel to improve efficiency. A higher number of patients may be processed thus increasing patient throughput, thus answering an ever-pressing need with an aging population in parts of the world. However, despite being autonomous, in order to decrease patient stress level, some tele-communication means may still be provided for the patient so they can contact human staff, in case of emergency, either real or perceived.

The medical procedure, such as an imaging session, is governed by a protocol P. For example, an imaging protocol P may prescribe a sequence of applicable control parameters *C* to be applied at the respective equipment Ej at certain times, and may further call for a list of medical action points (*aᵢⱼ*) that may be required from the patient to be perform when at, or interacting with, the respective equipment Ej. As said, the action points may include moving from one equipment Ej to another Ej+1, and/or may include performing certain one or more actions a_{i,j} when at a certain equipment Ej or interacting therewith. For present purposes, the action points to be performed by the patient may be considered as an ordered list (*aₖ*) =(a_{1,1}....a_{N},₁; a_{1,2},... a_{M,2}, ...) , whether or not geographical movement from one place to another is required.

The medical imaging protocol P may thus be understood as a list of a patient -associated component (*aₖ*) and a machine machine-associated component cj. The patient- associated component prescribes the sequence of actions (*aₖ*) to be performed by the patient at certain times, whilst the machine-associated component (*cₖ*) includes a list of applicable control parameters to control for example voltage/ amperage of the x-ray tube XS. The machine-associated component (*cₖ*) may specify a radio pulse sequence to be applied in an MRI imager, or may describe control settings of associated components, such as table ET movement, tube XS movement, collimator blade adjustments, etc. The patient-associated component and/or machine-associated component (*cₖ*) may include timing information.

More formally then, the imaging protocol P may be written as *P=[(cₖ), (aₖ)]* where each set of one or more control parameters *(cₖ)* is associated with one or more action points *(aₖ)* to be performed by the patient at sequence position *k*, when associated control parameter(s) *cₖ* is applicable in respect to the current medical equipment *E*ⱼ. The control parameters *cₖ* have a timing association *k* with the medical action points *a*ₖ, and may be applied before, concurrently, or after completion of the respective action point *aₖ* at sequence position *k* of protocol *P*. It will be understood that the notation "*c*ₖ" may be understood as a vector *c*ₖ =(*c*_{*1*,*k*}, *c*_{*2*,*k*}, ..., *c*_{*L*,*k*}) that describes plural setting parameters for different aspects or different types of equipment to be used as the same time/sequence position *k*. The protocol P may be formalized, represented and stored as a data file, preferably in structured form. For example, the file representing the medical protocol to be used for the current medical procedure may be coded in a markup language, such as an XML file for example, with nodes representing the specific action points and associated machine control parameters. Other data structures such as associative arrays, pointers or others still me be used instead or in addition.

The concept of patient-associated action points (*aₖ*) as used herein is to be construed broadly as it may require the patient to be passive rather than active. For example, an action point may require the patient to remain motionless in a certain posture from a certain time point for a period of time. In other instances, the action point may require a patient to actively perform a certain movement, assume a certain posture (moving one or more limbs, rolling over left/right, turning head, opening one's mouth, etc), or may call for the patient to operate certain parts of the equipment through actuators (pressing buttons, pulling levers etc), or interact physically in an otherwise prescribed manner with the equipment Ej. In addition or instead, the medical action point (*aₖ*) may ask the patient to breath at a certain frequency, faster or slower, or may indeed requisition a breath hold for a certain period, etc.

Performing such a medical action points in the right order and correctly may not be easy, in particular in the above-mentioned autonomous settings. There may thus be an undue burden on the patient to remember all the medical action points (*aₖ*) to be performed in the right order and at the right time. On the other hand, if the medical action points (*aₖ*) are not all performed correctly, the result of the procedure, such as the acquired imagery, may be non-diagnostic/ useless. Such a result may be undesirable as this may require re-running the medical procedure, adding costs and time delays for other patients etc. Repetitions of the same imaging session due to incorrect protocol may also put undue burden on the medical equipment and this may lead to premature wear-and-tear. For example, cathode discs in x-ray imagers are subjected to very high temperature gradients which can lead to cracks. Premature wear-and-tear on cathodes discs may thus call for replacement and associated imager down-time. Implementing the protocol P right first time may thus foster longer up-times of the equipment, thus lowering the risk of such premature wear and tear effects, and so promoting better imaging throughput for example.

In order to better assist the patient to implement the medical protocol P correctly and/or to reduce premature wear and tear on medical equipment Ej, a computer implemented user guidance system UGS is proposed herein. The user guidance system UGS may synthesize, as will be explained in more detail below, a media sequence M that is tailored to the patient's preferences and the medical protocol P to be performed. The media sequence, such as a motion picture (video), an audio feature such as a re-told story, etc, or a sequence of still images, a combination of two or more of the foregoing, is synthesized by the user guidance system. The synthesized media sequence M may be replayed/played back during and/or prior to the medical procedure. As will be explained in more detail herein, the generated media sequence such as video footage includes embedded landmarks that correspond to the action points required by the patient in the correct order. Landmarks are chosen according to user's media preferences to promote memory retention. It is assumed that participants are more likely to remember instructions if embedded into a narrative vector of their respective preference and inclination.

As proposed herein, the category or type of the media sequence so generated is based on user preferences. The media synthesized sequence may be conceptualized as a memory map that includes the said landmarks to guide the patient through the medical procedure, be it at one and the same equipment and/or moving from one equipment to another performing different action points there. The so synthesized media sequence may be used to render footage on a display device in form of a "movie" featuring the preferred landmarks so chosen, configured and embedded so as to echo the requisite action points in the right order. For example, the footage may feature scenes by a patient-preferred-actor who is shown to perform the right type of actions according to the protocol to so encourage memory retention and the patient's joining in and "playing along". High levels of sustained patient compliance may be achieved this way.

The media sequence may be consumed by patient PAT before the start of the medical procedure, in a preparatory or instruction phase when the media sequence is played back by suitable transducer equipment TR. In addition or instead, the media sequence may be consumed during the medical procedure, by playback of the media sequence, possibly suitably synchronized based on, for example, the above mentioned surveillance system, such as cameras that monitor patient movement, actions, posture, etc, during the medical procedure. The use of the synthesized media sequence during an on-going medical procedure may be referred to herein as the real time embodiment, whereas the former may be referred to as the off-time embodiment. Replaying of the synthesized media sequence M may be implemented in either embodiment for example by using mobile terminal devices, such as a smart phone or other portable data processing devices. The device may include a display device DD for example on which the replay of the media sequence is displayed, thus preparing the user, or cueing him or her what to do. The portable device may include instead or in addition to the display device DD, a loudspeaker to generate audio. A video, audio or multimedia representation of the media sequence is generated during the playback operation. An augmented/virtual reality system (AR/VR) may be used for the playback operation. In such AR/VR replay systems, footage may be synthesized that represents for example the patient's preferred actor performing the requisite medical action point. The action point as per the actor is fused with clinical surroundings that represent the equipment with which the user is expected to interact, thus and mimicking the action point.

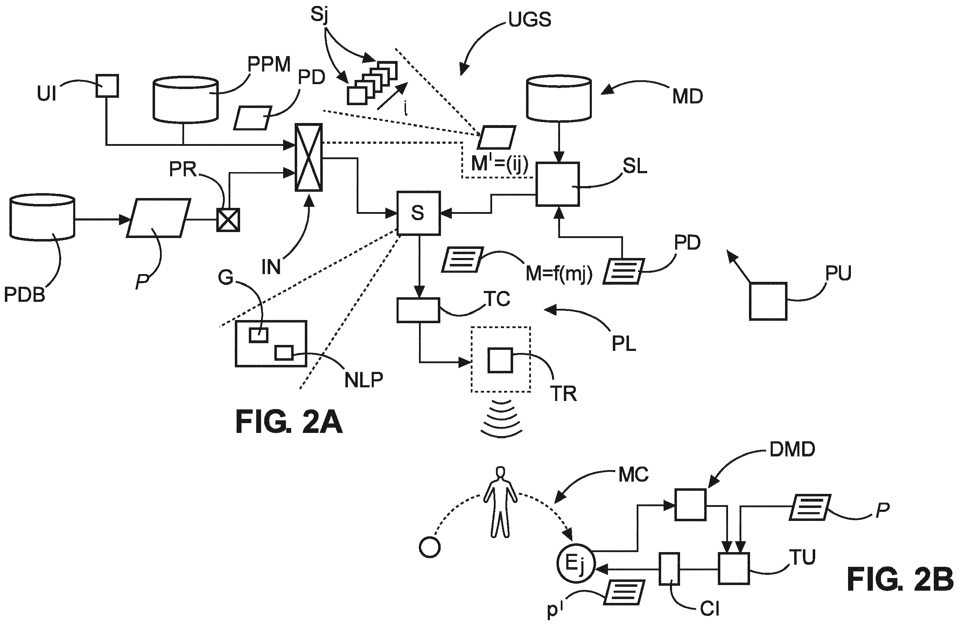

The user guidance system UGS may thus be understood to comprise two components, a synthesizer component that synthesizes the media sequence adapted to protocol and patient preference, and a playback component that uses the synthesized media sequence to generate a video, audio or multimedia stream. The playback component and/or synthesizer component may be implemented as an application ("app"), to run on the user's device for example, their smart phone, tablet computer, etc. However, fixedly installed playback devices with suitable transducers such display(s), loudspeakers, VR/AR headsets etc, suitably arranged and distributed across the medical facility, such as at the imaging or other applicable type of equipment Ej, is also envisaged herein. The user guidance system UGS may be implemented on one or more computing devices PU. Operation of the system UGS will now be explained in more detail with reference to the schematic block diagram in Fig. 2.

The user guidance system UGS includes a synthesizer component S. In some embodiments, operation of synthesizer component S is based on prior media data from which the new media sequence M, the memory map, is synthesized. Specifically, synthesizer component S (referred to herein simply as the "synthesizer S") receives at one or more if its input interface IN, at least parts of the protocol and patient preference data. In some embodiments where prior media is used, a selector SL is operative to select an initial media sequence M' from a media database MD. The synthesizer S processes the data from the protocol P, in particular the action point sequence (*aₖ*)*,* and the initial media sequence to synthesize the new media sequence as a memory map tailored to the patient preference data. The newly synthesized media sequence M may be a video file that represents the required patient action points as corresponding scenes. Alternatively, the media sequence is an audio file representing a sequence of story elements that in turn represents the requisite action points in the right order as per the protocol P.

The memory map M may be passed onto a player PL component configured to replay the memory map to the patient. The player PL component transforms the video or audio-based memory map into a perceptible signal stream on which the patient PAT can act on, thus facilitating successful participation by the patient in the medical procedure. Specifically, based on the media sequence M, transducer control circuitry controls a transducer TR to cause the user perceptible signal stream. The transducer TR may include a visual transducer, such as display device and/or an audio transducer, such as loudspeaker system. For example, if the media sequence is an audio file, a suitably modulated sound stream may be generated which the user will perceive as his or her favorite story being re-told, with audio scenes adapted to reflect the respective action points. In visual embodiments, a video feed may be displayed on the display device (as a visual transducer) to visually guide the patient through the requisite action points as per the medical procedure, again with scenes adapted based on the requisite action points, for example with actors in scenes performing acts that correspond to the action points.

The memory map may be synthesized based on a synthesizer function *ƒ* from prior existing media elements *mⱼ* of the initial media sequence M'. The initial media sequence M' was selected based on user preference data, such as user's favorite movie. A given media element *mⱼ* may represent an individual video or audio frame, or a group of such frames. Generally, some or each media element *m*ⱼ may represent a scene that corresponds to the action point (*aₖ*)ⱼ₌ₖ that the patient is expected to perform at the given time *j* in the correct order, relative to the other scenes that represent different, preceding or follow-up action points (*aⱼ*)_{*j*>*k orj*<}*ₖ.* Thus, in simple embodiments, the synthesizer function *f*, M =*f(*(*m*ⱼ)*)*, may be a permutation function π(*j*) -> *j*', M =(*m_{j'}*)*.* The memory map M, once generated, may be passed through a suitable communication link to player PL where the user guidance signal is generated as described above.

At least two principal modes of operation of the user guidance system UGS are envisaged herein in embodiments: in one mode it is the patient who initiates the processing. The patient may merely provide their media preference for the processing to start. The protocol is then obtained automatically by the system UGS from protocol database PDB, where the intended protocol for the patient is stored in association with a patient identifier. In another mode, the processing is "system-sided", so is initiated by the medical facility (such as a hospital for example). For example, a clinical user may feed an intended protocol P into the user guidance system to initiate the processing. It is now the user preference data that may be obtained automatically by system UGS.

In general, the input data received at input interface IN includes the patient preference data PD. This data may indicate a desired category of the initial media sequence M' preferred by the patient. This patient preference data may be queried from a memory PPM, where patient preference data PD is stored. The memory PPM may for instance be part of an end user device, such as patient's mobile phone, smart phone or other hand-held or tabletop device. The user guidance system may request the preference data through the interface IN, prior to user consent. Alternatively, social engineering may be used to access the patient's social media account or network (provided patient consent is given), to automatically infer the user preference for the media category. Access by system UGS may be restricted to posted public data.

The patient preference data OD may include in particular said category of the media sequence from which the memory map is to be synthesized. For example, the category may indicate a movie type/genre (spy, comedy, drama, etc), a favorite actor, director or other data that describes the preferred media sequence which the patient prefers. Alternatively, instead of system UGS actively sourcing ("pulling") the patient preference data from a memory PPM, a user interface UI, such as a touch screen or other may be provided for the patient to operate, so that patient can "push" the data for processing by system UGS. The patient can thus actively select a certain category of media sequence preference such as the preferred movie genre, featured actors(s), director, etc. In other embodiments, the user interface may be interfaced to a movie or audiobook database, and the user selects via the interface the said specific movie or audiobook. Alternatively still, the user uploads an electronic copy of the movie or audiobook file through interface unto the system UGS.

In terms of audio files, the user preference may indicate a literary genre, for example a crime, fiction, non-fiction, period, author, etc. An audio book file, or an indication of a preferred audio book file may be included in the patient preference data PD.

The input receivable at interface IN may further comprise at least in parts the medical protocol P as may be obtained from the protocol data base PDB. The medical protocol P encodes in particular the sequence of action points (*a*ₖ). At this point, it is only the action point sequence that is required, and not the associated control parameters, such as the radio pulse sequencing to be used in an MRI imaging session for example, or control parameters for a radiation therapy apparatus, etc as mentioned above. In other words, only the patient executable action points (*a*ₖ) part of the protocol P may be provided at this stage.

For example, a parser PR may be used. The parser PR parses the protocol file P to be used in the upcoming medical procedure in respect of patient PAT. The parser PR extracts the sequence of medical action points (*a*ₖ) in the correct sequence from the protocol file. The action points (*a*ₖ) may be defined in terms of natural language or may be otherwise encoded in an agreed coding scheme.

The synthesizer S receives the sequence of action points (*a*ₖ) based on protocol data *P*, and may receive an initial media sequence M' of the category as indicated by the user preference data PD. The synthesizer component analyzes the initial media sequence M' to identify a sequence of scenes (Sj) as represented by the sequence of media components (mj) that form this initial sequence M'. Scenes (Sj) are then identified in the initial media sequence M' that correspond to the sequence of patient action points (*a*ₖ). The patient action encoding sequence (*a*ₖ) thus forms master data, with slave data being the identified scenes (Sj). The action points (*a*ₖ) form anchor points to locate respective, corresponding scenes (Sj) in the initial media sequence M', and the so located scenes may be reordered (permuted) as per the action point sequence (*a*ₖ).

In general then, the media elements are adapted to the prescribed sequence of action points (*a*ₖ). For example, in one embodiment the synthesizer includes a natural language processing pipeline NLP. The initial media sequence M' may be provided for example as an audio file, representative of a story selected by patient. For example, the story may be retold by the patient and is recorded as the said audio file. The provided audio file represents the patient preference data PD. Alternatively a patient specified audio file representing the patient preferred story is sourced from the media data base MD. The audio file may be provided in form of an audiobook for example. The category of the audio file may include story title, genre, author, etc, and is as indicated in the patient preference data. If only a broad category is indicated, such as "crime story", the synthesizer may request through suitable retrieval interfaces a random story that falls under the preferred category. Patient's agreement to the random selection may be sought, and, if rejected, another random story is selected, and so forth, until patient indicates agreement. A similar random selection for broad categories may be implemented for video files ("movies").

The audio file may be transformed into text form by a speech-to-text module. Natural language processor NLP then analyzes the text to identify text portions that correspond to the action points. A suitable text similarity metric may be used to identify for some each action point, a corresponding text portion. The identified text portions (the slave data) are ordered according to the action point sequence master data. The reordered text portion may then be transformed back into audio-file to obtain the new media sequence/memory map M adapted to the protocol action points. A transformation between text and audio may not necessarily required, and the analysis may be done on the audio elements themselves, using a phonetic speech recognition. Suitable encoding techniques such as vector quantization ("VQ") codebooks may be used to define a suitable similarity metric.

A similar analysis based on image recognition may be done for a video sequence for example by identifying suitable scenes in the initial movie file M'.

The natural language processing pipeline or the image recognition component of the synthesizer S may be implemented by suitably trained machine learning models for example.

In case the initial analyzed media initial sequence M' does not include a suitable scene that can be associated with a required action point *aₖ*, certain stock or standard elements may be introduced instead to compliment the memory map M. The previously prepared stock elements may be held in the media data base MD in a suitable library.

In particular, in embodiments where the system UGS initiates the synthesizing of the memory map M, the user preference PD is automatically determined as indicated above by requesting data from the user's personal device through a suitable API. Based on the so automatically established user preference, the user guidance system UGS accesses the media database MD and identifies the initial media sequence video/audio that corresponds to the automatically determined preference data PD.

The adaptation of the individual medial elements (mj) that form the respective scenes (Sj) of the memory map may include more than a mere re-ordering/permutation. In addition or instead, suitable modulations of sound and/or changes to the color balance may be used to implement a patient priming mechanism, preferably during replay. For example, the color cast of certain scenes when displayed on screen can be changed to indicate that the patient is currently acting according to action points (*aₖ*) of protocol P. For example, a gradual or abrupt color cast operation such as a red shift may be effected to indicate the patient does currently not act in conformance with the protocol, whilst a green shift color cast may indicate the patient is doing the right thing. In addition or instead, audio modulation of the replayed audio file (story) or of the audio track of the replayed video M may be affected for priming purposes. Such audio modulation may include changes of tempo, rhythm, a shift in melody mode/pattern (dissonance vs consonance), etc. The patient may thus be encouraged to perform actions in line with the protocol.

The above-described embodiments of synthesizer S pre-suppose an initial media sequence M' from which the memory map M is synthesized, using the action points (*aₖ*) as master data. However, ab initio synthesizer embodiments are also envisaged herein, where there may be no reliance on such prior existing initial media sequence M'. Such ab initio embodiments may be preferably implemented by machine learning ("ML") models, preferably of the generative type. For example, generative adversarial networks ("GAN") have been proposed in the past that allow generating instances of a target distribution, in addition or instead, Autoencoder ("AE") type networks may be used. GANs and AEs are specially configured neural network ("NN") type ML models or systems thereof. Specifically, convolutional NNs ("CNN") may be used. Preferably the NN is of a multi-scale architecture including not only convolutional operators but also deconvolutional operators. U-net type architectures may be used in embodiments. CNN type NNs are useful when processing image data, such as video footage as envisaged herein for synthesized media sequence of the video type. The network may be of the feedforward type, but is preferably of the recurrent to better process time series data such as video.

A bank of such machine learning models of the generative type may be pre-trained, one (or more) for each for the different category of media sequence from which the user may wish to choose. For example, one model may be trained for movies of a certain genre (spy, action, comedy, etc), whilst other one or more models are trained based on motion pictures featuring a certain actor and/or directed by a certain director, etc. Other training date may include sport sequences, dancing sequences or travel sequences, travelogues, etc. The selector component SL then selects from the bank of pre-trained generator models the one that matches the category indicated by the patient preference data.

The generative ML modelling relies on a training data set for a given media category. A specially configured objective function (such as cost or utility function) guides an optimization procedure where parameters of the generative ML model are adapted in one or more training phases. Once trained and preferably tested, the model may be deployed in clinical practice a generator M of synthesizer S to produce memory map based on user preference. Only the category needs to be supplied by patient or system for the selector to select the trained model G from the bank. The trained generator G generates the memory map in the style of the patient specified category and based on the action point sequence by producing a corresponding sequence of scenes in the preferred category style. The selected generator G processes respective patient action points (*a*ₖ). The generator G generates for each such action point *a_{k'}* ∈ (*aₖ*)*,* a respective scene G(*a*_{k'})= *S*(*k'*), in the style corresponding to the patient selected category. In this manner, for given action point (eg, "*moving left arm up*", a scene may be generated featuring the preferred actor (as per the patient specified category), performing the action ("*moving left arm up*") the patient is expected to perform for example. Such techniques using generative models have been previously described as facilitators of "deepfake". It is proposed herein to use such deepfake ML techniques to generate, realistic, convincing user guidance media sequences (memory maps) that represent the correct sequence of action points by scenes in a diction that corresponds to the patient selected media category.

Turning first to AE type networks, such a network comprises an encoder part in serial combination with a decoder part. The encoder receives input data and transforms this into a simpler, in general lower dimensional representation. The encoder attempts to reconstruct at its output the input. The objective function measures a deviation of the two, and adjusts parameters of encoder and decoder until the decoder learns to reconstruct input from output within an acceptable error margin. Two such AE networks may be trained. One AE network is trained on video footage or imagery of the specified category (featuring a preferred actor for example). This network may be referred to as the "category" network, having its encoder (E^{c}) and decoder (D^{c}). The other network, the action point network, is trained on more general footage of different categories, each made up of scenes that represent the given action point from the list (*aₖ*)*,* again having its own encoder (E^{a}) and decoder (D^{a}) for the action point network. The desired scene for the memory map may then be synthesized ab initio by feeding a random input "seed" scene (*r*) into the encoder of the action network to obtain the associated latent representation which is now fed into the decoder of the category network to obtain the desired synthesized scene: Sⱼ = D^{c}(E^{a}(*r*)). This may be repeated for some or each action point in (*aₖ*) to so obtain all scenes Sj that make up the memory map. The bank of generators may thus have a matrix structure comprising AE networks for each action point *a* and each category *c*, each such network G^{a,c} having its own trained encoders and decoder.

Turning now to the embodiments of synthesizer S based on GAN type networks, GAN networks as such were previously described by Ian Goodfellow et al in "Generative Adversarial Nets", available on preprint server at arXiv:1406.2661v1, published 10 Jun 2014. GANs comprise two NN models, a discriminator network ("D") and a generator network G.

The discriminator D may be set-up as an NN-classifier. The discriminator D may be arranged as a deep fully connect network, a deep fully convolutional network, or as a hybrid network including one or more layers of both type.

In operation during training, the GAN set-up works as follows. The generator G generates from a seed signal (r) applied to it (such as random noise for example), a sample scene G(r). The discriminator D attempts to discriminate between the generator output G(r), and genuine, ground truth scenes as held in a training database. The ground truth comprises historic media sequences of the patient prescribed category that represents the action points. For example, the historic media sequences includes video footage scenes collected from motion picture databases that represent the patient's favored actor performing the target action points.

A specially configured cost function is used, based on which the parameters of generator G and discriminator D are adjusted. Such as cost function was described by *Goodfellow et al* at eq(1), page 3. The cost function represents two opposed objectives similar to a zero-sum-game: the objective in respect of the discriminator D is to maximize its probability of correct labelling, whilst the objective in respect of generator G is produce output so that the discriminator D cannot statistically discriminate between a real scene drawn from the training database, and generated scenes generated by the generator.

The parameters of the two models G,M may be adjusted in a min-max procedure in alternate manner in separate runs of iterations. The iterations cause the parameters of the models G,D to converge in a type of a Nash-equilibrium, at which point the generator is capable of generating realistic looking "fake scenes", featuring for example the patient preferred category, such as a preferred actor performing the target action point. At this point, the discriminator D is no longer capable for discriminating between fake and ground truth, and the discriminator D is such no longer needed. The generator G can thus be used to generate from a random seed signal (*r*), instances of target action scene in the same style as per the patient prescribed media category *c*. Again, different such GAN models may be trained for different categories and action points G^{a,c}, and stored in the bank. As before for the AE models, the selector SL may use appropriate index (aₖ, c) to access the correct GAN generator G^{a}ₖ^{,c} in the bank. The synthesizer S may then use the accessed model G^{a}ₖ^{,c} to ab-initio-generate the respective scene for index (*aₖ, c*)*.* Any of the above mentioned ab initio embodiments may be combined with any of the above mentioned synthesizing schemes based on prior initial media.

However, it will be understood that the above described ML based embodiments are not a necessity herein and are optional embodiments. It has been observed, that the earlier, simpler, not necessarily ML based embodiments of the synthesizer S already produces suitable memory maps that already have been found to have a positive impact on patient compliance.

The operational mode of the user guidance system UGS as described above was mainly based on the premise that the applicable imaging protocol represents the master data based on which in the slave data (that is the in initial media sequence) are adapted or generated by synthesizer. However, there may be a feedback loop envisaged herein, where it is the protocol data, in particular the control parameters (ck), that may be adapted based on the patient's action in respect of the medical procedure. Specifically, it is in particular the timing of the control parameters (*cₖ*) that is adapted. The control parameter timing relates to the time periods in between time points at which the control parameters are changed over from *cₖ,* to *c*_{*k*+*1*} as per the protocol. Whilst the synthesized media sequence/ memory map M is replayed, delays may be expected in relation to patient's actions. The delays indicate the respective time period that passes between when the respective scene *Sⱼ* is played out, and the time point when the user actually initiates or completes the action point *aⱼ* associated with that scene *Sⱼ*. This delay is patient specific and could be measured in a preparatory phase or real-time during the medical procedure itself.

To this end, the user guidance system UGS may include a delay measurement system DMD. This may be implemented as a camera or sound based surveillance system, with suitably placed cameras, microphones, or other suitable sensors, that monitor actions of the patient as mentioned earlier. A suitable image recognition component of the system DMD, possibly machine learning based, is operable to analyze the recorded surveillance data (eg, frames) that captures the actions of the user. The system DMD monitors for correspondence of the capture actions with the required action points (ak).

If such a delay is established, a tuner component TU adjusts the existing control parameters (*cₖ*) and/or their timings, so as to ensure synchrony with the patient caused delay. In this manner, an adapted imaging or radiation therapy protocol P' is generated, tailored to the delay habits of the patient. These delay habits may correlate with the patient's reaction and comprehension times. A suitable control interface CI may be used to control the respective imaging equipment or other medical equipment Ej based on the adapted protocol P'. Optionally, the media sequence M may be adjusted after delay measurement to achieve the original imaging protocol timing.

As briefly mention above, the player PL component may be embedded in an augmented or virtual reality system. For example a VR (virtual reality)/AR (augmented reality) headset may be used in which the memory map video is replayed for the patient.

In order to enhance memory retention still, the player system PL may be in communication with a haptics delivery system that induces certain haptic signals delivered to the patient in correspondence with the replayed memory map. For example, the examination table TE or the RF coil support of the MRI imaging system IA2 may be caused to vibrate. The timing, frequency or intensity of the vibration caused my correlate to the degree of patient compliance in respect to the requisite action points, and hence how well patient follows the instructions represented by the memory map.

In any of the above-mentioned embodiments, if the protocol sequence includes action points for the patient to physically move from one exam room to another, or from one equipment *Eⱼ* to the next Eⱼ₊₁, this part of the action points may be synthesized into a separate "geographical" memory map, with scenes from a travel video with landmarks representing different items of equipment. The map is hence representative of a journey that represents the medical produced at least in parts as a journey, a medical circuitry mapped into historic, cultural landmarks of one or more countries according to user preference. The action points at the respective equipment may then be synthesized as described above based on preferred media category. The geographical memory map may be combined with any of the earlier mentioned memory maps.

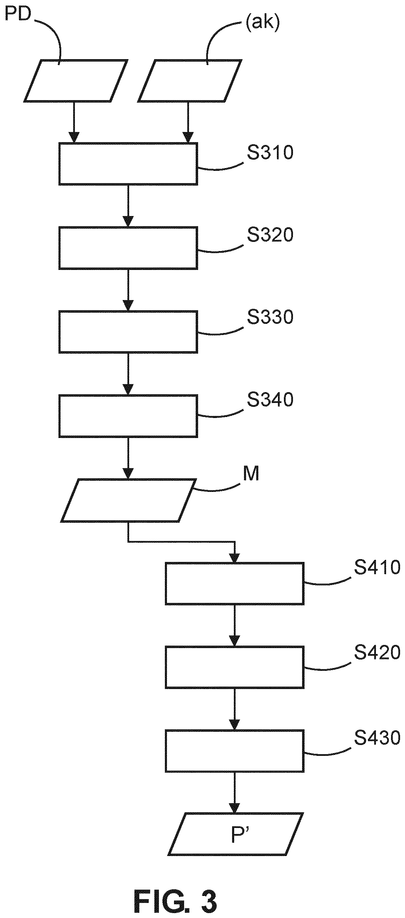

Reference is now made to the flow chart in Fig. 3 which illustrates steps of a computer implemented method for user guidance, based on media sequences synthesized as described above.

At step S310, a protocol for a medical procedure such as for an imaging procedure or radiation therapy is received. The protocol indicates medical action points to be performed by a patient to undergo the medical procedure.

At step S320 indication of a category of the be synthesized media sequence is received. The indication may be through patient input via a user interface. Thus, the category indication may be caused by the patient, by active involvement of the patient. Alternatively, no such active involvement on the part of the patient is required. For example, the data may be automatically retrieved by accessing personal preference data of the patient, such as may be held on a social media account of the patient or in a memory of a device of patient, such as their smartphone, tablet or other preferably portable device. To this end, a Bluetooth, NFC or other wireless connection may be established using suitable APIs.

At step S330, the target media sequence M (referred to herein as the memory map) is synthesized including media elements such as individual frames or groups of such frames. The media elements represent respective individual scenes. Each of the media elements in the synthesized media sequence are so synthesized that the represented scenes causable by replaying the media sequence correspond in the correct order to the requisite action points as per the protocol.

At step S340 a transducer, such as a display device and/or loudspeaker system of a replay device is controlled, to produce a patient perceptible guidance or instruction/tuition signal, based on the synthesized sequence. This signal may comprise for example video footage displayed on the display device in accordance with the media sequence and/or the signal may comprise sounding out an audio signal, such as re-telling of a story in accordance with the media sequence.

The media sequence for replay may be used for real time guidance of the user during an ongoing medical procedure being performed according to protocol. In addition or instead, the media sequence may be presented to the user in a preparation or instruction session prior to commencement of the medical procedure.

The memory map so generated may thus be understood to include certain landmark scenes, tailored around the patient's preference and functionally corresponding to the requisite action points in the correct order as per the imaging protocol.

The method may optionally comprise, in addition, or instead of steps S310-S340, an adaptation of the underlying imaging protocol, in particular of control parameters, such as imaging parameters or radiation therapy delivery parameters, and the timings thereof. The adaption may be based on certain delay periods incurred whilst the patient is reacting to the replayed media sequence.

Specifically, at step S410, the delay period is measured. The delay period comprises the time period between the presentation of a user perceptible signal to the patient of a given scene, and the user's performance of action point (ak) associated with the scene. The scene is represented by the media elements of the synthesized media sequence.

At step S420 the imaging protocol, in particular its control parameters and/or their timings, may be adapted based on the delay measurements.

The synthesized media may be adapted based on the delay information to optimize, or at least improve timing and hence the protocol. For example, for X-ray / CT there might be the option to delay switching the x-ray source XS on, to adapt the patient bed movement / positioning, etc. In MRI, the start of a pulse imaging sequence might be delayed or idle times could be adapted between such sequences, etc.

At step S430 the medical equipment, such as an imaging apparatus or radiotherapy delivery apparatus, is controlled based on the adapted imaging protocol P'.

The components of the user guidance system UGS may be implemented as one or more software modules, run on one or more general-purpose processing units PU such as a workstation associated with an imager IA1,2, or on a server computer associated with a group of imagers or other types of medical equipment.

Alternatively, some or all components of the user guidance system UGS may be arranged in hardware such as a suitably programmed microcontroller or microprocessor, such an FPGA (field-programmable-gate-array) or as a hardwired IC chip, an application specific integrated circuitry (ASIC). In a further embodiment still, the user guidance system UGS may be implemented in both, partly in software and partly in hardware.

The different components of the user guidance system UGS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A user guidance system (SYS) comprising:
an interface (IN) for receiving a specification of a medical protocol for a given patient, the specification defining a sequence of medical action points;
a synthesizer (S) configured to synthesize a media sequence in accordance with the sequence of medical action points, to obtain a synthesized media sequence for the said patient, the media sequence suitable to guide the patient in a medical procedure based on the medical protocol.

2. System of claim 1, wherein the media sequence is of a pre-selected category.

3. System claim 2, comprising a selector (SL), configured to select the category based on i) input from the patient or user received through a user interface (UI), and/or ii) based on characteristics of the patient.

4. System of any one of the previous claims, comprising a transducer controller (TC) configured to control, based on the synthesized media sequence, a transducer (TR) to produce a media output for the patient.

5. System of claim 4, wherein the synthesized media sequence comprises a sequence of media elements associated with respective ones of the action points.

6. System of claim 5, wherein the synthesizing operation includes: i) changing an order of media elements in an existing media sequence and ii) modulating a media element in accordance with an action point.

7. System of claim 5 or 6, comprising a delay measurement device (DMD) configured to measure an elapsed time period between the transducer being controlled as per a given media element and the patient preforming the associated action point.

8. System of claim 7, comprising a tuner module (TU), configured to tune the specification of the medical protocol based on the measured time period to produce a tuned specification of the medical protocol.

9. System of claim 8, comprising a control interface, configured to control a medical imaging apparatus or therapy apparatus, based on the tuned specification.

10. System of any one of the previous claims, wherein the media sequence is one or more of: audio, imagery and video.

11. System of any one of the previous claims, wherein the synthesizer is based on a machine learning model of the generative type.

12. An arrangement, comprising a medical imaging or therapy device and the system as per any one of the previous claims.

13. A computer-implemented method, comprising:
receiving (S310) a specification of a medical protocol for a given patient, the specification defining a sequence of medical action points; and
synthesize (S330) a media sequence in accordance with the sequence of medical action points, to obtain a synthesized media sequence for the said patient, the media sequence configurable to guide the patient in a medical procedure, based on the protocol.

14. A computer program element, which, when being executed by at least one processing unit, is adapted to cause the processing unit to perform the method as per claim 13.

15. At least one computer readable medium having stored thereon the program element of claim 14.
